# EUROPEAN PATENT APPLICATION

(11) **EP 4 475 136 A1**
(43) Date of publication of application: **11.12.2024**
(21) Application number: 24180535.7
(22) Date of filing: 06.06.2024
(51) Int. Cl.: G16H 20/30, G16H 10/00

(54) **SYSTEM AND METHOD FOR IMPROVING THE TRAINING EXPERIENCE OF A USER**

(30) Priority: 07.06.2023 IT 202300011637
(71) Applicant: Technogym S.p.A., 47521 Cesena, Forli'-Cesena (IT)
(72) Inventor: CEI, Daniele, I-47521 Cesena, FORLI'-CESENA (IT)
(74) Representative: Mozzi, Matteo

(57) **Abstract**

A system (100) for determining and storing training data of a plurality of users (P-U) in a training space (A), comprising at least one central data processing unit (1), at least one central memory unit (2) operatively connected to said at least one data processing unit (1), and at least one image acquisition device (4) present inside the training space (A) and operatively connected to the at least one central data processing unit (1). The at least one image acquisition device (4) is configured to communicate data with the central data processing unit (1) and to acquire images of said plurality of users (P-U) within the training space (A). Each user of said plurality of users (P-U) is assigned with a respective user identification code (ID1-IDN) stored in the at least one central memory unit (2). The at least one central data processing unit (1) is configured to perform, for each user of said plurality of users (P-U), for each current time instant ti, with 1 < i < N, of a plurality of consecutive time instants t₁, t₂, ..., t_{N}, with N being an integer, the following operations:
- a1) determining, when the user performs the physical exercise identified for the user within a set training program, information (I-E) representative of training data of the user related to the physical exercise being performed by the user;
- b1) storing, in the at least one central memory unit (2), said information (I-E) representative of training data of the user related to the physical exercise being performed by the user;
- c1) determining, when the user performs a physical exercise identified for the user within the set training program and stored in the at least one central memory unit (2), at least one of: a value (V-TP) representative of the time elapsed since the user started performing the physical exercise, a value (V-PS) representative of the position of the user within the training space (A), a value (V-TR) representative of a training parameter provided by the physical exercise being performed by the user;
- d1) identifying a next physical exercise for the user to be performed within the training space (A) provided by the set training program, based on the respective user identification code (ID1-IDN) and at least one of: whether or not the determined value (V-TP) representative of the time elapsed since the user started performing the physical exercise reaches a respective time target provided by the physical exercise being performed by the user, the determined value (V-PS) representative of the position of the user within the training space (A), whether or not the determined value (V-TR) representative of a training parameter provided by the physical exercise being performed by the user reaches a respective training target provided by the physical exercise being performed by the user;
- e1) storing, in the at least one central memory unit (2), the next physical exercise identified for the user;
- f1) providing the user with an indication of the next physical exercise identified for the user;
- g1) repeating steps a1) - f1) for the next physical exercise identified for the user.

## Description

### Field of the invention

The present invention relates to the field of fitness, and in particular, to a system and a related method for improving the training experience of a user.

### Technological background of the invention

The tracking of the training data of a user is a very important aspect for improving the training experience of the user.

In fact, the data of a training being performed or performed by a user on an exercise machine, on which the user had previously authenticated to perform the assigned training program, are stored, for example, in a remote electronic computer or cloud, so as to update the profile of the user and to allow, at a subsequent authentication on an exercise machine, the user him or herself to continue the training program resuming from the last stored profile, and/or a personal trainer, given the results obtained so far by the user, to possibly modify the training program of the user.

It is apparent that the traceability and storage of the training data of a user are facilitated by the fact that an exercise machine is remotely connected to the remote electronic computer or cloud and by the fact that the user, before starting the training on a specific exercise machine, authenticates in advance so as to set the profile thereof and the respective training program.

However, a user, within a training program, can perform not only exercises which involve the use of one or more exercise machines but also or exclusively bodyweight exercises and/or exercises which involve the use of more simple equipment, such as kettlebells, dumbbells, weights and so on, which obviously could not allow a connection to the remote electronic computer, nor a user authentication.

In this case, the user would have to upload the exercises performed by hand via an electronic device connected to the remote electronic computer, with a waste of time and considerable difficulty in remembering all the exercises performed.

All this limits the traceability and storage of user data and user training data which would therefore risk not corresponding to the actual physical activity performed, therefore being outdated and unreliable for improving the training experience of the user.

A technological evolution aimed to overcome such a limit consists in monitoring the training space with cameras so as to acquire in real time digital images of a user while training, even bodyweight training, and obtain such training data from the processing of the digital images acquired.

Such a processing, in addition to requiring sophisticated digital image processing algorithms, is quite expensive from a computational point of view, especially if the images acquired, even by multiple cameras, refer to a large number of users who are training simultaneously in the same training class.

In the light of this, precisely to improve the training experience of a user as much as possible, the need is strongly felt to be able to determine, and therefore to track, and store the training data of a user during any exercise performed by a user, whether on or with an exercise machine, a bodyweight exercise or using any other equipment available so as to be able to track and store the training data of a user which are as complete as possible, thus avoiding as much as possible the need to resort to onerous and expensive computational resources.

### Summary

It is the object of the present invention to devise and provide a system for determining and storing training data of a plurality of users in a training space which allows at least partially obviating the abovementioned drawbacks with reference to the prior art, in particular which is capable of performing such operations during any exercise performed by a user, whether on or with an exercise machine, a bodyweight exercise or using any other equipment available so as to be able to track and store the training data of a user which are as complete as possible, thus avoiding as much as possible the need to resort to onerous and expensive computational resources.

Such an object is achieved by a system according to claim 1.

Preferred embodiments of said system are defined in the dependent claims.

The present invention also relates to a method to be carried out with such a system.

### Brief description of the drawings

Further features and advantages of the system and the related method according to the invention will become apparent from the following description of preferred embodiments thereof, given by way of non-limiting indication, with reference to the accompanying drawings, in which:
- Figure 1 shows, by means of a block diagram, a system for determining and storing training data of a plurality of users in a training space, in accordance with an embodiment of the present invention;
- Figure 2 shows, by means of a block diagram, a system for determining and storing training data of a plurality of users in a training space, in accordance with a further embodiment of the present invention;
- Figures 3a-3f each show, by means of a respective block diagram, the system in Figure 1 in accordance with a respective embodiment;
- Figure 4a diagrammatically shows an example of an exercise machine which can be used in the system in Figure 1 in accordance with an embodiment of the invention;
- Figure 4b diagrammatically shows a further example of an exercise machine which can be used in the system in Figure 1 in accordance with an embodiment of the invention;
- Figure 4c shows from a functional point of view, by means of a block diagram, an exercise machine which can be used in the system in Figure 1 in accordance with an embodiment of the invention;
- Figure 5a diagrammatically shows an example of an exercise tool which can be used in the system in Figure 1 in accordance with an embodiment of the invention;
- Figure 5b diagrammatically shows a further example of an exercise tool which can be used in the system in Figure 1 in accordance with an embodiment of the invention;
- Figure 5c shows from a functional point of view, by means of a block diagram, an exercise tool which can be used in the system in Figure 1 in accordance with an embodiment of the invention;
- Figure 6 diagrammatically shows, by means of a block diagram, a method for determining and storing training data of a plurality of users in a training space, in accordance with the present invention;
- Figure 7 shows, by means of a time diagram, an example of a set training program for a plurality of users which can be used in the system and in the method according to the present invention, and
- Figure 8 shows, by means of a table, a further example of a set training program for a plurality of users which can be used in the system and in the method according to the present invention.

It should be noted that, in the aforesaid figures, equivalent or similar elements are indicated by the same numeric and/or alphanumeric reference.

### Detailed description

With reference to the aforesaid Figures, a system 100 for determining and storing training data of a plurality of users in a training space is described, hereinafter also referred to as a system for determining and storing training data only or simply a system, in accordance with the present invention.

"Training space", indicated below and in the Figures with alphanumeric reference A, means any indoor or outdoor space, comprising one or more areas separated from one another by walls or other similar separation elements and communicating with one another by means of doors or other equivalent access elements, in which a plurality of users can train, in which one or more of the electronic components included in the system 100, described below, are present or can be reached.

Examples of a training space are an indoor or outdoor gym, an outdoor space, an indoor home space, an outdoor home space (for example, a balcony, a terrace, a courtyard), and so on.

"Training data of the user" means data representative of a physical exercise performed by a user of the plurality of users P-U within the training space A such as, for example, data relating to the movement of the user during the physical exercise (for example, posture while running or cycling, and so on, if the physical exercise performed by the user involves the use of a respective exercise machine, or number of squats, number of sit-ups, number of lunges, number of push-ups, and so on, if the physical exercise performed by the user is a bodyweight exercise), operating parameters of an exercise machine used by the user to perform the physical exercise; data relating to the movement of the exercise machine, or parts thereof, used by the user to perform the physical exercise (even if associable with the data relating to the movement of the user, movement of the levers of an elliptical machine, movement of a handle of a rower, movement of the levers of a strength machine, movement of a weight stack of a strength machine, and so on); data relating to an exercise tool (for example, weight, shape, color, and so on); data relating to the movement of an exercise tool (for example, even if associable with the movements of the user, for example, data relating to the movement of a weight, a dumbbell, a barbell, a kettlebell, and so on).

A definition of "exercise machine" and a definition of "exercise tool" will be provided below.

"Determining and storing training data of a plurality of users in a training space" means the detection and/or traceability and/or calculation and subsequent storage of the aforesaid data during the stay of a user of the plurality of users P-U within the training space.

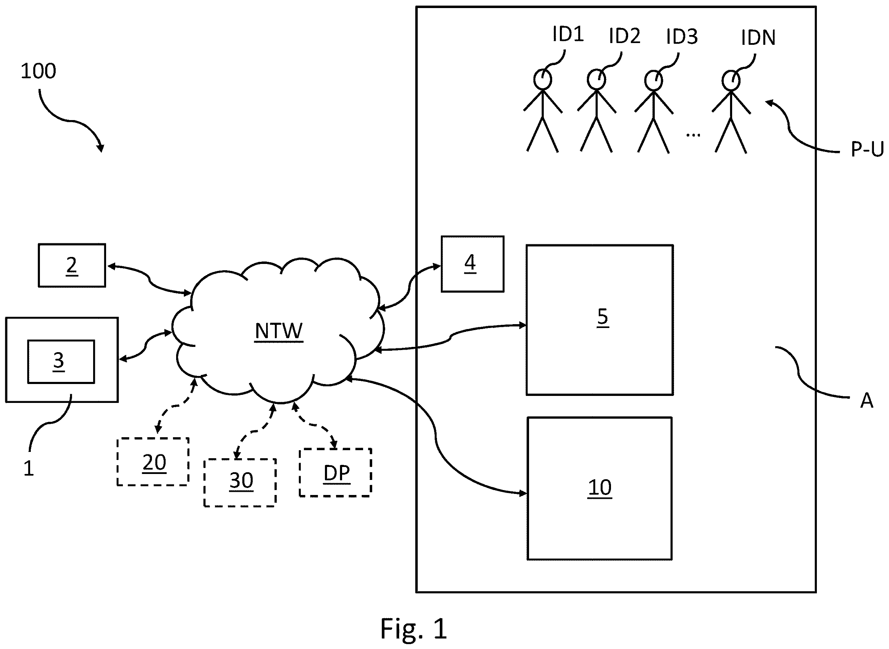

With particular reference to Figure 1, the system 100 comprises at least one central data processing unit 1, for example a microcontroller or a microprocessor.

The system 100 further comprises at least one central memory unit 2 operatively connected to the at least one central data processing unit 1.

The at least one central data processing unit 1 is configured to load and perform one or more program codes (previously stored in a respective local memory unit 3, diagrammatically shown in Figure 1) to perform respective functions including determining and storing training data of a plurality of users P-U in a training space A, as it will be described below.

It should be noted that the system 100 can comprise one or more central data processing units 1, and one or more respective central memory units 2, so as to be capable of carrying out the respective functions, depending on the configuration, totally locally, i.e., inside the space A, totally remotely with respect to the space A, or in a hybrid mode, i.e., with functions to be carried out locally and functions to be carried out remotely, with respect to the space A.

Returning to Figure 1, the system 100 further comprises at least one image acquisition device 4 present inside the training space A and operatively connected to at least one central data processing unit 1.

The at least one image acquisition device 4 is configured to communicate data with the at least one central data processing unit 1.

The at least one image acquisition device 4 is configured to acquire images of said plurality of users P-U within the training space A.

The at least one image acquisition device 4 can be any electronic device which allows acquiring digital images and/or digital videos inside the space A, whether it is a gym (indoor or outdoor gym) or a home space (indoor or outdoor home space).

Examples of image acquisition devices are a 2D digital camera, a 3D digital camera, an RGB camera, an RGB-D camera, a video camera, a depth camera, a Time-Of-Flight (TOF) camera, a webcam, a thermal camera, a motorized camera, and so on.

The image acquisition device can have specific technical features including resolution (for example: 5MP, 8MP, 16MP, 1080p, 4K or else), focal length (for example: wide angle, short telephoto, medium telephoto, super telephoto or else), aperture range (for example: f/1.8, f/4, f/16 or else), field of view FoV (for example: 90°, 120°, 180° or else), aspect ratio (for example: square, portrait, landscape, panoramic or else), frame ratio (for example: 30fps, 50fps, 60fps or else), type of focus (for example: auto-focus, manual focus, fixed focus or else), installation type (integrated, built in, embedded, standalone).

It should be noted that "image acquisition device present inside the training space A" means that the at least one image acquisition device 4 is available inside the training space A so that it can acquire images of each user of the plurality of users P-U during the stay thereof inside the training space A.

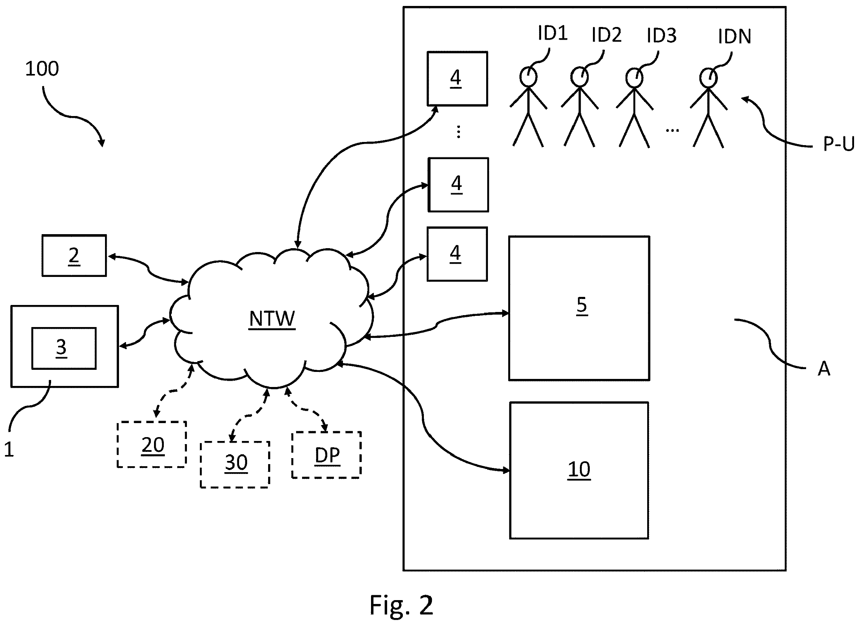

In an embodiment, in combination with the preceding ones and shown, for example, in Figure 2, the system 100 can comprise a plurality of image acquisition devices 4, completely similar to the at least one image acquisition device 4 described above.

The presence of more than one image acquisition device 4 advantageously allows the system 100 to better follow the plurality of users P-U inside the training space A, being thus able to improve the reliability and accuracy of the system 100 itself.

The more general case in which the system 100 comprises said at least one image acquisition device 4 can certainly relate to when the training space A is the gym, but above all when the training space A is the home space, where it is more likely that only one of the aforesaid devices is present.

Returning to the present invention, each user of said plurality of users is assigned with a respective code ID1, ID2, ID3, ..., IDN.

The identification code is stored in the at least one central memory unit 2.

In accordance with the present invention, the at least one central data processing unit 1 is configured to perform, for each user of said plurality of users P-U, for each current time instant tᵢ, with 1 < i < N, of a plurality of consecutive time instants t₁, t₂, ..., t_{N}, with N being an integer, the following operations.

The at least one central data processing unit 1 is configured to a1) determine, when the user performs the physical exercise identified for the user within a set training program, information I-E representative of training data of the user related to the physical exercise being performed by the user.

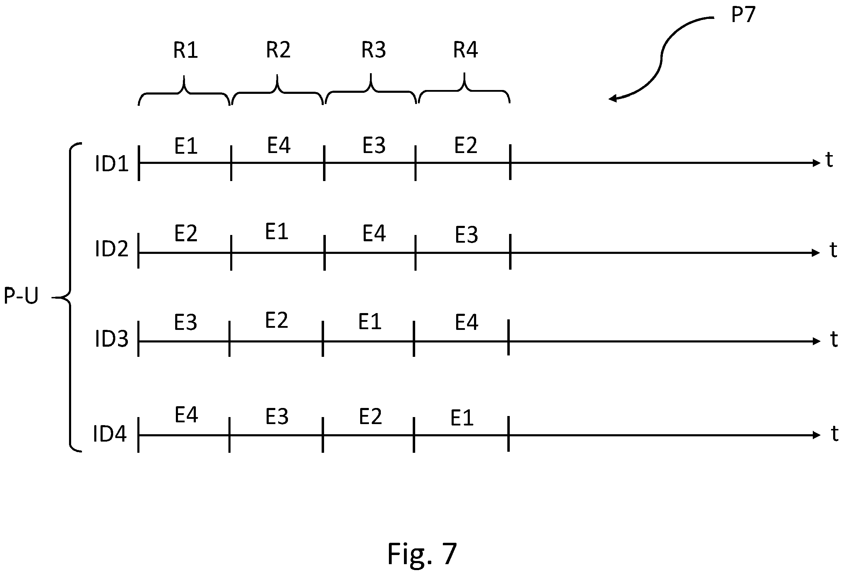

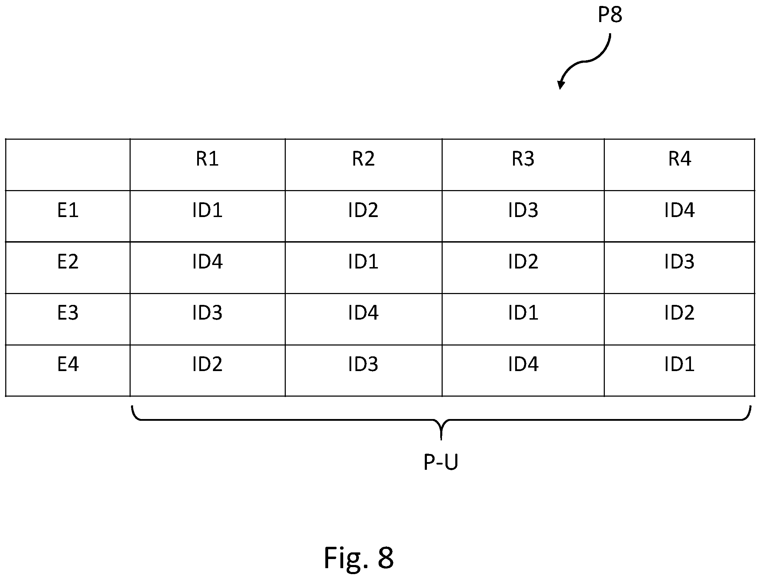

Examples of the set training program will be described below with reference to Figures 7 and 8.

The at least one central data processing unit 1 is configured to b1) store, in the at least one central memory unit 2, said information I-E representative of training data of the user related to the physical exercise being performed by the user.

The at least one central data processing unit 1 is configured to c1) determine, when the user performs a physical exercise identified for the user within the set training program and stored in the at least one central memory unit, at least one of: a value V-TP representative of the time elapsed since the user started performing the physical exercise, a value V-PS representative of the position of the user within the training space A, a value V-TR representative of a training parameter provided by the physical exercise being performed by the user.

In more detail, the value V-TP representative of the time elapsed since the user started performing the physical exercise is determined by the at least one central data processing unit 1, for example by means of a timer.

The value V-PS representative of the position of the user within the training space A is determined by the at least one central data processing unit 1.

In one embodiment, the value V-PS representative of the position of the user within the training space A is determined by the at least one central data processing unit 1 by means of a processing of the images of the user within the training space A acquired by the at least one image acquisition device 4.

In a further embodiment, alternatively to or in combination with the preceding one, the value V-PS representative of the position of the user within the training space A is determined by the at least one central data processing unit 1 by means of localization techniques based on multilateration if the use of a short-range wireless communication protocol in ultra wideband technology is provided by the at least one central data processing unit 1 and other electronic devices present in the training space, as it will also be reiterated below.

The value V-TR representative of a training parameter provided by the physical exercise being performed by the user, if the physical exercise being performed by the user involves the use of an exercise machine or an exercise tool (for example, a dumbbell) which is not configured to automatically detect and communicate training data, or if the physical exercise being performed by the user is a bodyweight exercise (for example, sit-ups, lunges, push-ups, and so on), is determined by the at least one central data processing unit 1 by means of a processing of the images of the user within the training space A acquired by the at least one image acquisition device 4.

It should be noted that "exercise machine not configured to automatically detect and communicate training data" means an exercise machine whose configuration, in terms of equipment of electrical and/or electronic components, does not allow the detection of training data and the automatic communication of detectable training data for the purposes of implementation of the present invention.

For example, the exercise machine may not be configured to detect training data as it does not have training data detection sensors.

For example, the exercise machine may not be configured to communicate training data as it does not have a data communication interface (wire, wireless or other equivalent technology).

For example, the exercise machine may be configured to detect training data and communicate such training data only subsequently the use of the exercise machine, or the exercise machine may be unable to communicate training data, or the exercise machine may be configured to detect training data and not communicate any training data.

It should be noted that "exercise tool not configured to automatically detect and communicate training data" means an exercise tool without any electrical and/or electronic component, therefore an exercise tool without any possibility of detecting training data, much less to communicate such training data, or an exercise tool whose configuration of the respective electrical and/or electronic components does not allow the automatic communication of detectable training data for the purposes of implementing the present invention.

For example, the exercise tool may not be configured to detect training data as it does not have training data detection sensors.

For example, the exercise tool may not be configured to communicate training data as it does not have a data communication interface (wire, wireless or other equivalent technology).

Therefore, the exercise tool may not be able to automatically detect and communicate training data as, in general, it lacks any electrical and/or electronic components or it may be configured to detect training data and to communicate such training data only after the use of the exercise tool, or the exercise tool may be unable to communicate training data, or the exercise tool may be configured to detect training data and not communicate any training data.

If the physical exercise being performed by the user involves the use of an exercise machine or exercise tool (for example, a dumbbell) which is not configured to automatically detect and communicate training data, such a training parameter can be, for example, the number of repetitions performed on the exercise machine or with the exercise tool.

If the physical exercise being performed by the user is a bodyweight exercise, such a training parameter can be, for example, the number of squats, the number of sit-ups, the number of lunges, the number of push-ups, and so on.

The value V-TR representative of a training parameter provided by the physical exercise being performed by the user, if the physical exercise being performed by the user involves the use of an exercise machine or an exercise tool configured to automatically detect and communicate training data, is determined by the at least one central data processing unit 1 by means of an analysis of the received training data.

Such a training parameter can be, for example, the meters traveled on a treadmill, the meters traveled on a bike, the number of calories consumed, the number of repetitions performed on the exercise machine, and so on.

The at least one central data processing unit 1 is configured to d1) identify a next physical exercise for the user to be performed within the training space A provided by the set training program, based on the respective user identification code ID1-IDN and at least one of: whether or not the determined value V-TP representative of the time elapsed since the user started performing the physical exercise reaches a respective time target provided by the physical exercise being performed by the user, the determined value V-PS representative of the position of the user within the training space A, whether or not the determined value V-TR representative of a training parameter provided by the physical exercise being performed by the user reaches a respective training target provided by the physical exercise being performed by the user.

To "identify" the next physical exercise for each user to be performed within the training space A provided by the set training program means identifying, by the at least one central data processing unit, the next physical exercise within a set sequence (or prescription) of physical exercises to be performed in sequence provided for each user within the set training program previously stored in the at least one central memory unit 2, which the at least one central data processing unit 1 can consult based on the quantities indicated above (user identification code and at least one of position, time target and training target).

In more detail, the at least one central data processing unit 1 is configured to compare the determined value V-TP representative of the time elapsed since the user started performing the physical exercise with the respective time target provided by the physical exercise being performed by the user and, once the time target provided by the physical exercise being performed by the user is reached, knowing, for example, the sequence of physical exercises of the set training program, it is capable of identifying the next physical exercise for the user.

In greater detail, by way of example, it should be noted that the set training program, stored in the central memory unit 2, can provide, for each user of the plurality of users P-U, a division of the training time into set training time intervals, each comprising a physical exercise.

Therefore, as it will also be indicated below, each set training time interval can be associated with an exercise machine (rower, treadmill, bike, and so on) or an exercise tool (dumbbell, kettlebell, and so on) or a station for bodyweight training.

The at least one central data processing unit 1 thus knows the respective time sequence of the physical exercises of each user provided by the set training program.

Each time target provided by the physical exercise being performed by the user is the time limit at which the set training time interval provided for the respective physical exercise ends.

Therefore, once the respective time target provided by the physical exercise being performed by the user has been reached, the latter being identifiable by the respective user identification code, the at least one central data processing unit 1 is capable of identifying the next physical exercise for the user and indicating to the user the next physical exercise to perform.

Alternatively or in combination, the at least one central data processing unit 1 is configured to compare the determined value V-PS representative of the position of the user within the training space A with corresponding position values of a preset configuration of the training space A, stored in the central memory unit 2.

In greater detail, by way of example, it should be noted that the preset configuration of the training space A, stored in the central memory unit 2, provides for a division of the training space A into set areas.

The determined value P-S representative of the position of the user within the training space A corresponds to a set area of the training space A identifiable in such a preset configuration.

In this case, as it will also be indicated below, each set area of the training space A can be associated with an exercise machine (rower, treadmill, bike, and so on) or an exercise tool (dumbbell, kettlebell, and so on) or a station for bodyweight training.

Therefore, having determined the set area in which a user is located, thus the physical exercise being carried out by the user, the latter identifiable by the respective user identification code, the at least one central data processing unit 1 is capable of identifying the next physical exercise for the user.

For example, when the user has reached the training time target provided for the physical exercise being performed by the user or the training target provided by the physical exercise being performed by the user associated with a specific training parameter, the at least one central data processing unit 1 is capable of indicating to the user the next physical exercise to perform.

Alternatively or in combination with one or more of the preceding cases, the at least one central data processing unit 1 is configured to compare the determined value V-TR representative of a training parameter provided by the physical exercise being performed by the user with the respective training target provided by the physical exercise being performed by the user and, once the training target provided by the physical exercise being performed by the user is reached, knowing, for example, the sequence of physical exercises of the set training program, it is capable of identifying the next physical exercise for the user.

In greater detail, by way of example, it should be noted that the set training program, stored in the central memory unit 2, can provide, for each user of the plurality of users P-U, a sequence of physical exercises, each of which is associated with a respective provided training target.

This sequence can comprise the use of an exercise machine (rower, treadmill, bike, and so on) or an exercise tool (dumbbell, kettlebell, and so on) or a bodyweight training.

Therefore, the at least one central data processing unit 1 also knows the respective sequence of the physical exercises of each user provided by the set training program.

Therefore, once the respective training target provided by the physical exercise being performed by the user has been reached, the latter being identifiable by the respective user identification code, the at least one central data processing unit 1 is capable of identifying the next physical exercise for the user and indicating to the user the next physical exercise to perform.

Generally returning to the invention, once the next physical exercise for the user has been identified, the at least one central data processing unit 1 is configured to e1) store in the at least one central memory unit 2 the next physical exercise identified for each user of the plurality of users P-U.

The at least one central data processing unit 1 is configured to f1) provide the user with an indication of the next physical exercise identified for the user.

If the identification of the next physical exercise occurs based on the fact that the determined value V-TP, representative of the time elapsed since the user started performing the physical exercise, reaches the respective time target provided by the physical exercise performed by the user, the at least one central data processing unit 1 provides the user with the indication of the next physical exercise identified for the user and the user, once aware of such an indication, will reach the station and will actually move on to perform the next physical exercise.

If the identification of the next physical exercise occurs based on the determined value V-PS, representative of the position of the user within the training space A, the at least one central data processing unit 1 provides the user with the indication of the next physical exercise identified for the user. In this case, the user, once aware of such an indication, should already be in the position corresponding to the next identified physical exercise which should also already be performed by the user. If this is not the case, such an indication provided to the user helps the user to understand which physical exercise they should actually perform in that position within the training space A.

If the identification of the next physical exercise occurs based on the fact that the determined value V-TR, representative of a training parameter provided by the physical exercise being performed by the user, reaches the respective training target provided by the physical exercise performed by the user, the at least one central data processing unit 1 provides the user with the indication of the next physical exercise identified for the user and the user, once aware of such an indication, will reach the station and will actually move on to perform the next physical exercise.

In accordance with any of the embodiments described above in which the at least one central data processing unit 1 provides the user with an indication of the next physical exercise identified for the user, such an indication can be provided, for example, by means of a video and/or audio message and/or an update of the status of the training class viewable on a display screen made available to the plurality of users within the training space A.

For example, the state of the training class can be a time diagram (like the one shown in Figure 7) or a matrix (like the one shown in Figure 8) in which each user can intuitively understand, by simply observing the display screen, which physical exercise (E1-E4) has been identified for the user (ID1 - ID4) to be performed within a training round (R1-R4) provided by the set training program (P7 in Figure 7; P8 in Figure 8).

Returning to the present invention, the at least one central data processing unit 1 is configured to g1) repeat steps a1) - f1) for the next physical exercise identified for the user.

In accordance with an embodiment, in combination with any of the preceding ones in which the presence of an exercise machine is provided and shown in the Figures, the system 100 further comprises at least one exercise machine 5 present within the training space A.

For the purposes of the present invention, "exercise machine" means any exercise machine comprising at least one actuator (motor and/or brake) which is operable (automatically or manually) while performing the physical exercise such as, for example, a treadmill, a bike, a rower, a strength machine with automatic parameter detection.

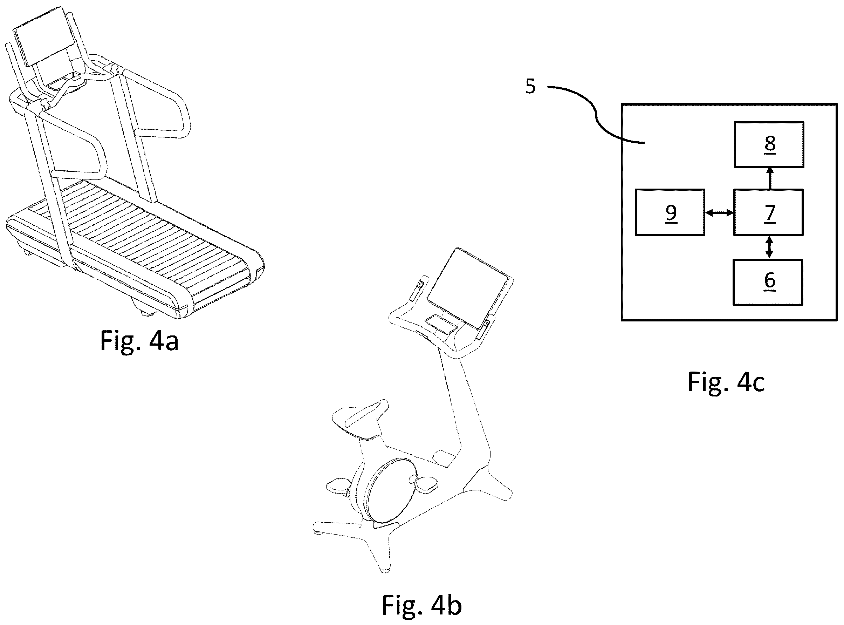

Examples of an exercise machine are diagrammatically shown and indicated without distinction by reference numeral 5 in Figures 4a (treadmill) and 4b (bike).

In one embodiment, the at least one exercise machine 5 is configured to automatically detect and communicate training data to the at least one central data processing unit 1.

In this regard, the at least one exercise machine 5 is operatively connected to the at least one central data processing unit 1.

According to different embodiments, the at least one exercise machine 5 is operatively connected to the central data processing unit 1 in a wired or wireless mode.

In an embodiment, diagrammatically shown in Figures 1, 2, 3a-3f, the at least one exercise machine 5 is operatively connected to the central data processing unit 1 by means of a data communication network NTW, for example, the Internet, diagrammatically shown in the Figures.

In greater detail, the data communication network NTW can be a local network, for example a local area network (LAN), or a telecommunications network which extends over a large geographical distance, for example a Wide Area Network (WAN) or a combination of one or more of the aforesaid types.

In a further embodiment, alternatively to or in combination with the preceding one, the at least one exercise machine 5 is operatively connected to the central data processing unit 1 in a wireless mode by means of a wireless technology data communication channel such as, for example, a Wi-Fi, Bluetooth, NFC data communication channel, and so on.

In accordance with different embodiments, the communication of the training data between the at least one exercise machine 5 and the central data processing unit 1 can occur by means of the data communication network NTW and/or a wireless technology data communication channel (Wi-Fi, Bluetooth, NFC, and so on).

In accordance with an embodiment, diagrammatically shown in Figure 4c, the at least one exercise machine 5 comprises at least one actuator 6 which is operable while performing the physical exercise.

The actuator 6 is, for example, an electric motor and/or a brake device being operable (automatically or manually by the user) while performing the physical exercise.

Still with reference to Figure 4c, the at least one exercise machine 5 further comprises a respective local data processing unit 7.

The actuator 6 is operatively connected to the local data processing unit 7.

Moreover, the at least one exercise machine 5 further comprises a local memory unit 8 operatively connected to said local data processing unit 7.

The at least one local memory unit 8 can be internal or external (as shown, for example, in Figure 2) with respect to the at least one local data processing unit 7.

It should be noted that the at least one local memory unit 8 is configured to store one or more program codes executable by the at least one local data processing unit 7 to generally control the at least one exercise machine 5 and, specifically, to perform respective functions provided within the operation of the system 100, as it will be described below.

Still with reference to Figure 4c, the at least one exercise machine 5 further comprises a data communication unit 9.

The at least one exercise machine 5, in particular the local data processing unit 7, is configured to communicate data to the central data processing unit 1 by means of the data communication module 9.

In accordance with different embodiments, the data communication module 9 is configured to communicate data by means of the data communication network NTW and/or by means of a wireless technology data communication channel (Wi-Fi, Bluetooth, NFC, and so on).

In accordance with an embodiment, as an alternative to the preceding ones, the at least one exercise machine 5 is not configured to automatically detect and communicate training data.

The definition of "exercise machine not configured to detect and automatically communicate training data" has been previously indicated.

In an embodiment, in combination with any of the preceding ones and not shown in the Figures, the system 100 can comprise a plurality of exercise machines 5, each entirely similar, from a functional point of view, to the at least one exercise machine 5 described above with particular reference to Figure 4c and to possible embodiments.

The more general case in which the system 100 comprises said at least one exercise machine 5 can certainly relate to when the training space A is the gym, but also to when the training space A is the home space, where it is more likely that only one of the aforesaid exercise machines is present.

In accordance with an embodiment, in combination with any of the preceding ones in which at least one exercise tool is present and shown in the Figures, the system 100 further comprises at least one exercise tool 10 present within the training space A.

"Exercise tool" precisely means any exercise tool free from any actuator (for example, motor and/or brake) which is operable (automatically or manually by the user) while performing a physical exercise such as, for example, a barbell, a dumbbell, a kettlebell, an elastic band, parallel bars, a strength tool with weight stack, a strength tool with disc holder levers (of the plate loaded type).

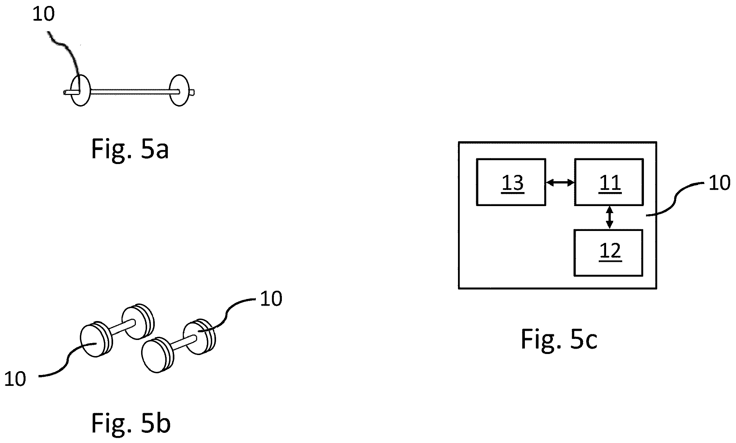

Examples of an exercise tool are diagrammatically shown and indicated without distinction by reference numeral 10 in Figures 5a (barbell) and 5b (pair of dumbbells).

The at least one exercise tool 10 is free from any actuator which is operable while performing a physical exercise.

In this case, the at least one exercise tool 10 is configured to be recognized within an image, for example, by means of the respective shape, color or a respective identification tag.

The at least one exercise tool 10 is characterized by one or more parameters representative of said at least one exercise tool 10.

Examples of such parameters are weight, spring constant or else, depending on the exercise tool 10.

In one embodiment, the at least one exercise tool 10 is configured to automatically detect and communicate training data.

In this embodiment, the at least one exercise tool 10 is configured to be operatively connected to the central data processing unit 1.

In accordance with an embodiment, the at least one exercise tool 10 is operatively connected to the central data processing unit 1 in a wireless mode.

In an embodiment, the at least one exercise tool 10 is operatively connected to the central data processing unit 1 by means of the data communication network NTW already described above.

In a further embodiment, alternatively to or in combination with the preceding one, the at least one exercise tool 10 is operatively connected to the central data processing unit 1 in a wireless mode by means of a wireless technology data communication channel such as, for example, a Wi-Fi, Bluetooth, NFC data communication channel, and so on.

In accordance with different embodiments, the data communication between the at least one exercise tool 10 and the central data processing unit 1 can occur by means of the data communication network NTW and/or a wireless technology data communication channel (Wi-Fi, Bluetooth, NFC, and so on).

In an embodiment, diagrammatically shown in Figure 5c, the at least one exercise tool 10 comprises a local data processing module 11, for example a microprocessor or a microcontroller.

In this embodiment, the at least one exercise tool 10 further comprises a local memory module 12 operatively connected to said at least one local data processing module 11.

The at least one local memory module 12 can be internal or external (as shown, for example, in Figure 2) with respect to the at least one local data processing module 11.

It should be noted that the at least one local memory module 12 is configured to store one or more program codes executable by the at least one local data processing module 11 to perform respective functions during the use of the at least one exercise tool 10, even during the operation of the system 100.

In an embodiment, in combination with the preceding one and shown with dashed lines in Figure 1, the at least one exercise tool 10 further comprises a local data communication module 13 operatively connected to the local data processing module 11.

The at least one exercise tool 10, in particular the local data processing module 11, is configured to communicate data to the central data processing unit 1 by means of the local data communication module 13.

In accordance with different embodiments, the local data communication module 13 is configured to communicate data by means of the data communication network NTW and/or by means of a wireless technology data communication channel (Wi-Fi, Bluetooth, NFC, and so on).

In one embodiment, as an alternative to the preceding ones, the at least one exercise tool 10 is not configured to automatically detect and communicate training data.

The definition of "exercise tool not configured to detect and automatically communicate training data" has been previously indicated.

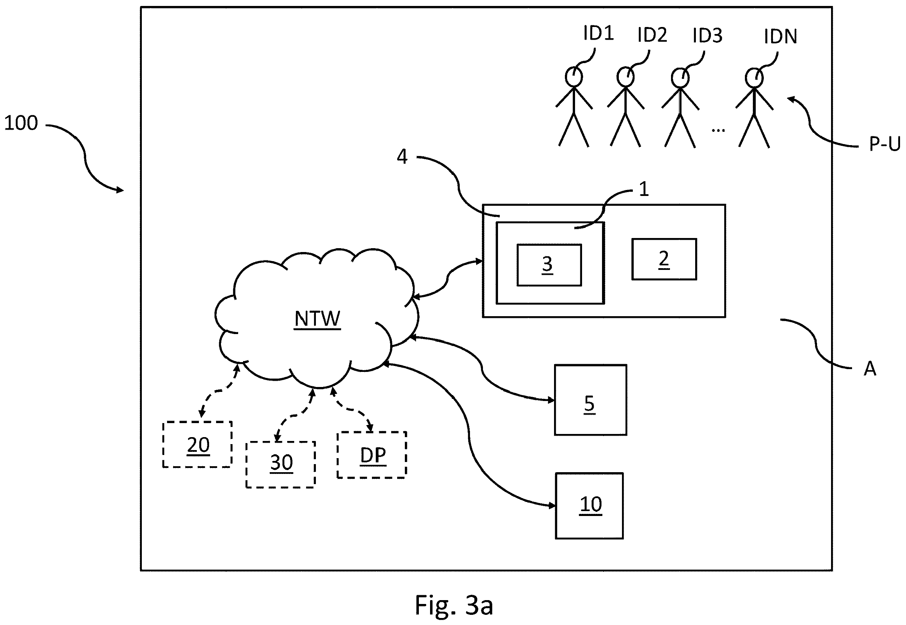

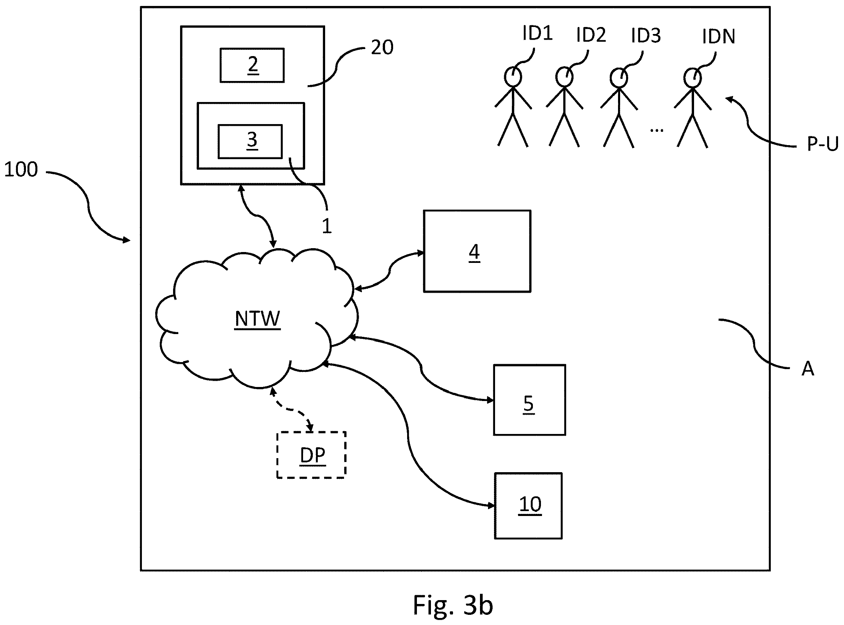

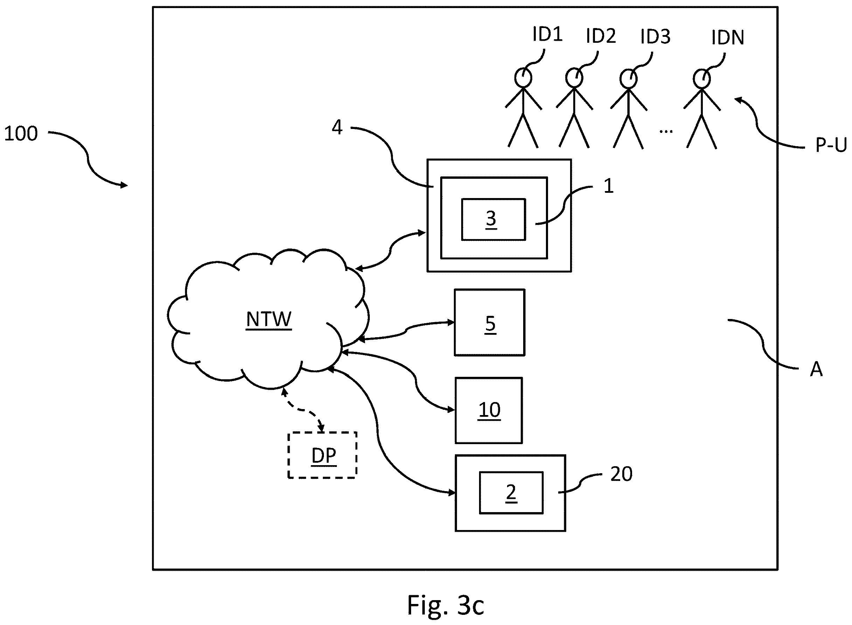

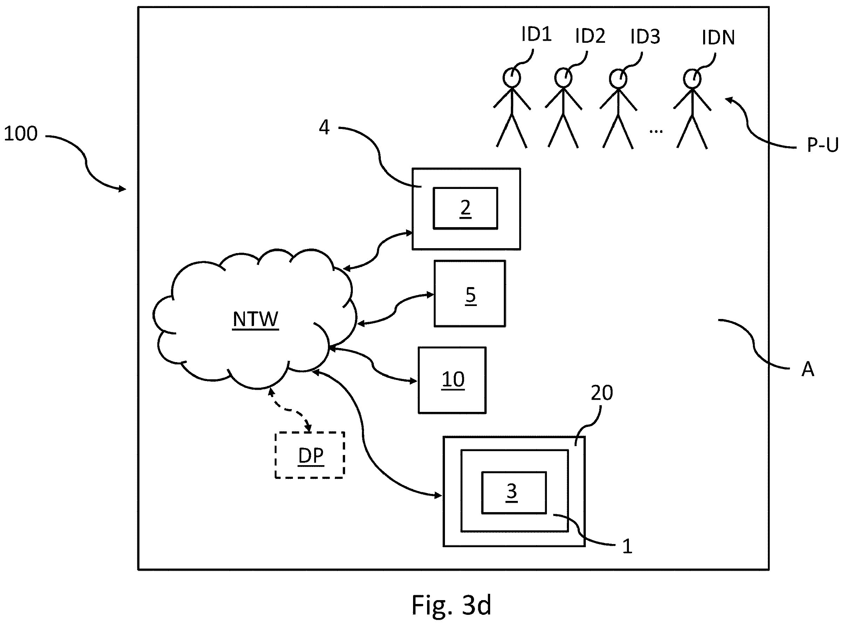

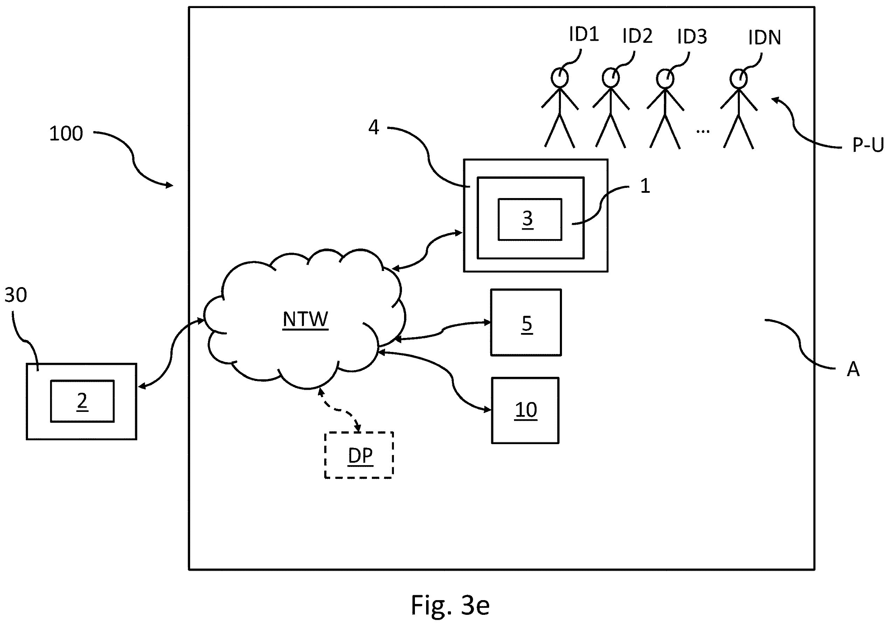

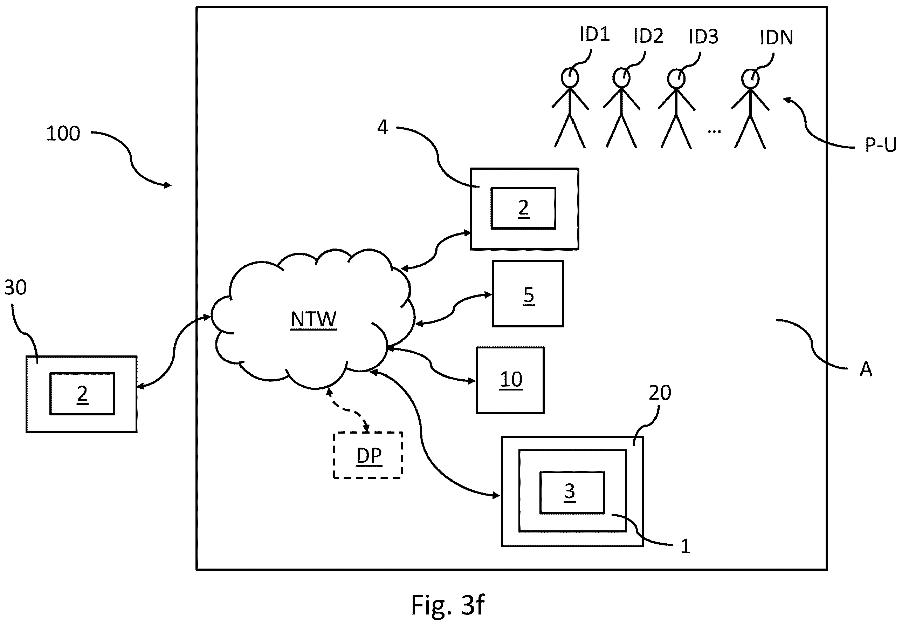

In an embodiment, in combination with the preceding ones and shown, for example, in Figure 3, the system 100 can comprise a plurality of exercise tools 10, each of which entirely similar, from a functional point of view, to the at least one exercise tool 10 described above with particular reference to Figure 5c and possible embodiments.

The more general case in which the system 100 comprises said at least one exercise tool 10 can certainly relate to when the training space A is the gym, but also to when the training space A is the home space.

In accordance with an embodiment, in combination with any of the preceding ones in which the presence of at least one exercise machine 5 or of the at least one exercise tool 10 is provided, the identified physical exercise being performed by the user involves the use of an exercise machine or exercise tool (for example, a dumbbell) which is not configured to automatically detect and communicate training data, or in which the identified physical exercise being performed by the user is a bodyweight exercise.

In this embodiment, the at least one central data processing unit 1 is configured to determine the information I-E representative of training data of the user related to the physical exercise being performed by the user based on a processing of the images of the user within the training space A acquired by the at least one image acquisition device 4.

It should be noted that the images of the user within the training space A acquired by the at least one image acquisition device 4 are a subset of the images of the plurality of users P-U within the training space A acquired by the at least an image acquisition device 4.

In other words, the at least one central data processing unit 1, by processing the images of the plurality of users P-U within the training space A acquired by the at least one image acquisition device 4 is configured to determine the users present within the training space A, recognizing for each of them the respective user identification code ID1-IDN and associating to each of them the respective identified physical exercise as provided by the set training program based on the respective prescription for that specific user identification code.

As it will also be reiterated below, the at least one central data processing unit 1 is capable of processing a diagrammatic model representative of the user (structure with a plurality of key elements (points) such as nodes and segments) and the respective training data (for example, it can count the repetitions performed).

In this respect, the at least one central data processing unit 1 is configured to perform such a determination by using a set motion tracking algorithm which in turn uses "computer vision" techniques, artificial intelligence algorithms, and so on.

By way of example, the at least one data processing unit 1, being the identified (prescribed) physical exercise for the user known, using artificial intelligence algorithms, is configured to recognize and classify a representative schematic model of the user (structure with a plurality of key elements (points) such as nodes and segments), detect the movements of the plurality of key elements (points) of the schematic model representative of the user to count the repetitions performed by the user, count the series performed by the user, assess the pace of the user, assess the range of motion (ROM), analyze the posture of the user while performing the exercise, segment the body of the user, recognize the body of the user, recognize the gestures of the user, recognize the face of the user (facial recognition), detect the movement of the user, detect the heart rate of the user, detect the fatigue felt by the user, detect the gender and/or race of the user, analyze the body mass index (BMI).

Examples of artificial intelligence algorithms can be Ordinary Least-Squares Regression (OLS), Multiple Linear Regression (MLR), Principal Component Regression (PCR), Partial Least-Squares Regression (PLS), Ridge regression (RR), Lasso Regression, Multivariate Adaptive Regression Splines (MARS), Stepwise Regression (SR), Nonlinear Regression, and many others.

In addition, classification techniques can also be used such as, for example, linear discriminant analysis (LDA), logistic regression (LR), classification and regression trees (CART), Gaussian mixture models (GMMs), k-nearest neighbors (k-NNs), artificial neural networks (ANNs), deep neural networks (DNNs), convolutional neural networks (CNNs), support vector machines (SVMs), partial least-squares-discriminant analysis (PLS-DA), multilayer perceptron classifiers (MLPs), radial basis functions (RBFs), and so on.

It should be noted that the techniques described above are usable by at least one central data processing unit 1 to perform other functions, described below in accordance with various embodiments, provided in the implementation of the system 100.

In accordance with an embodiment, in combination with the preceding one, the identified physical exercise being performed by the user involves the use of an exercise machine or exercise tool configured to automatically detect and communicate training data.

In this embodiment, the at least one central data processing unit 1 is configured to determine the information I-E representative of training data of the user related to the physical exercise being performed by the user based on an analysis of the training data received from the exercise machine or exercise tool.

In accordance with an embodiment, in combination with any of the preceding ones, the user identification code ID1-IDN of each user of said plurality of users P-U comprises respective user identification data.

"Identification data of a user" means data being usable used by the system 100 to identify the user, for example, data being obtainable from a code associated with the user (for example, a label or a tag associated with a garment, a color of a garment or of a bracelet), data being obtainable from a portable electronic device wearable by the user (of the wearable type), data being obtainable from a facial recognition of the user or from a recognition of the biometric parameters of the user (proportions of the lower limbs, upper limbs, torso, and others), and so on.

The identification data of the user comprise information on the identity of the user which, for privacy reasons, cannot be attributable to the user.

In accordance with an embodiment, in combination with any of the preceding ones, the at least one central data processing unit 1, for each user of said plurality of users P-U, for each current time instant tᵢ, with 1 < i < N, of the plurality of consecutive time instants t₁, t₂, ..., t_{N}, with N being an integer, is configured to determine, from a processing of first user identification images acquired during an initial user authentication step, a schematic reference model representative of the user.

Such a schematic reference model comprises a plurality of key elements (points) (nodes and/or segments) with which to uniquely represent the user.

In accordance with an embodiment, in combination with any of the preceding ones, the at least one central data processing unit 1, for each user of said plurality of users P-U, for each current time instant tᵢ, with 1 < i < N, of the plurality of consecutive time instants t₁, t₂, ..., t_{N}, with N being an integer, is configured to determine, from the processing of the images of the user acquired by at least one image acquisition device 4, a schematic model representative of the user while performing the identified physical exercise.

Such a schematic model comprises a plurality of key elements (points) (nodes and/or segments) with which to uniquely represent the user.

In this embodiment, the at least one central data processing unit 1 is configured to associate such a schematic model representative of the user while performing the identified physical exercise with the respective user identification code ID1-IDN.

In this embodiment, the at least one central data processing unit 1, for each user of said plurality of users P-U, for each current time instant tᵢ, with 1 < i < N, of the plurality of consecutive time instants ₁, t₂, ..., t_{N}, with N being an integer, is configured to determine the variation over time of the schematic model representative of the user while performing the identified physical exercise.

Such a variation over time of the schematic model representative of the user while performing the identified physical exercise comprises information representative of the movement of the plurality of key elements (points) of the schematic model representative of the user while performing the identified physical exercise.

Moreover, in this embodiment, the at least one central data processing unit 1 is configured to compare the information representative of the movement of the plurality of key elements (points) of the schematic model representative of the user while performing the identified physical exercise with set reference movement patterns stored in the at least one central memory unit 2 relating to the physical exercise identified for the user.

In greater detail, in the at least one central memory unit 2 a database is stored comprising, for each physical exercise of a plurality of physical exercises identifiable for each user of said plurality of users P-U (which can be set in accordance with a set prescription by, for example, a trainer or other training expert, as it will be reiterated below), a respective set reference movement pattern.

The at least one central data processing unit 1, being the physical exercise identified for the user known, is thus capable of querying the database stored in the at least one central memory unit 2 to recover the respective set reference movement pattern to be used for the aforesaid comparison.

Moreover, in this embodiment, the at least one central data processing unit 1 is configured to provide the user (for example, by means of an audio and/or video message) with an information representative of an assessment of the correct performance of the physical exercise by the user based on the outcome of such a comparison.

Therefore, it should be noted that, in this embodiment, the at least one central data processing unit 1 is capable of providing the user with the specific information representative of an assessment of the correct performance of the physical exercise by the user based on the monitoring of the variation over time of the plurality of key elements (points) of the schematic model without having to process any image acquired by the at least one image acquisition device 4, again with a notable saving from a computational point of view.

In this embodiment, the at least one central data processing unit 1, for each user of said plurality of users P-U, for each current time instant tᵢ, with 1 < i < N, of the plurality of consecutive time instants ₁, t₂, ..., t_{N}, with N being an integer, is configured to store in at least one central memory unit 2 the determined variation over time of the schematic model representative of the user while performing the identified physical exercise.

In accordance with an embodiment, in combination with the preceding one, the at least one central data processing unit 1 is configured to:
- determine a power built up by the user while performing the identified physical exercise within the training space A, the amplitude and speed of execution of the movements performed by the user determined by the processing of the images acquired by the at least one image acquisition device 4 (optionally, the weight of the user determined based on the identification images of the user U can also be used) and/or an energy consumed by the user (for example, number of calories) while performing the physical exercise;
- associate the determined built-up power and/or the determined built-up energy with the set reference movement patterns, if the movements performed by the user during the physical exercise correspond to said set reference movement patterns stored in the at least one central memory unit 2.

In accordance with an embodiment, in combination with any of the preceding ones in which the set reference movement patterns are present, said set reference movement patterns performed by the user are movements of the body of the user.

In accordance with an embodiment, in combination with any of the preceding ones in which the set reference movement patterns are present, said set reference movement patterns performed by the user are movements of said at least one exercise tool 10 moved by the user.

In this embodiment, for each user of said plurality of users P-U, for each current time instant tᵢ, with 1 < i < N, of a plurality of consecutive time instants t₂, tᵢ, ..., t_{N}, with N being an integer, the at least one central data processing unit 1 is configured to:
- determine a power built up by the user and/or an energy built up by the user which is a function of the identification images of the user and of said exercise tool 10 while performing a physical exercise;
- associate the determined built-up power and/or the determined built-up energy with the set reference movement patterns, if the movements performed by the user during the physical exercise correspond to set reference movement patterns stored in the at least one central memory unit 2.

In a further embodiment, in combination with any of those described above, the images acquired by the at least one image acquisition device 4 comprise the user identification code ID1-IDN (for example, garment color, label on garment, wearable device of the bracelet type...) and information representative of the posture and/or movements performed by the user.

In this embodiment, the at least one central data processing unit 1 is configured to extract, in real time, from the acquired images, and store in the at least one central memory unit 2 the user identification code ID1-IDN and the information representative of the posture and/or movements performed by the user U.

In a further embodiment, in combination with any of the preceding ones, shown with dashed lines in Figures 1, 2, 3a-3f, the system 100 further comprises a personal device DP wearable by one or more users of the plurality of users P-U.

In this embodiment, the at least one central data processing unit 1, for each user of said plurality of users P-U, is configured to acquire the user identification code from the personal device DP wearable by the user.

In this further embodiment, in combination with the preceding one, the at least one central data processing unit 1, for each user of said plurality of users P-U, is configured to acquire the user identification code from the personal device DP wearable by the user for each current time instant tᵢ, with 1 < i < N, of the plurality of consecutive time instants t₁, t₂, ..., t_{N}, with being N integer.

In general, the personal device DP wearable by the user is acquirable as an image by the at least one image acquisition device 4 and/or connectable in a wireless mode to the central data processing unit 1 and/or with the at least one image acquisition device 4, during the presence of the user in the training space A.

According to a further embodiment, in combination with or alternatively to the preceding one, the personal device DP wearable by the user is configured to be operatively connected in wireless mode with the at least one central data processing unit 1.

By way of example, the personal device DP wearable by the user is configured to be operatively connected in wireless mode to the at least one central data processing unit 1 by means of a short-range wireless communication protocol in ultra-wideband technology.

In more detail, ultra-wideband technology employs localization techniques based on multilateration, i.e., on the difference between the arrival time of a signal emitted by a mobile tag by a number of receiving stations (referred to as "anchors") located in a known area.

In this embodiment, the central data processing unit 1 is configured to detect the position of the user by using ultra-wideband technology, possibly exploiting the presence of multiple personal devices DP, each wearable by a respective user present within the training space A.

The at least one central data processing unit 1 is thus capable of determining the position of a user within the training space A, if the position of the user is used to identify the next physical exercise for the user, thus avoiding, from a hardware and computational point of view, the processing by the at least one central data processing unit 1 of the images acquired by the at least one image acquisition device 4.

Examples of a personal device which is wearable by the user are a bracelet, a wristband, a band, a smartphone, a smartwatch, or another electronic device which is wearable by the user, and so on.

In accordance with an embodiment, in combination with any of those described above, the at least one image acquisition device 4 is installed in the training space A.

For example, the at least one image acquisition device 4 can be mounted on a wall or on a post in a gym (outdoor or indoor), on a wall of a domestic space (outdoor or indoor), on an exercise machine, in the gym or in a home space).

In accordance with an embodiment, in combination with any of those described above and alternatively to the preceding one, the at least one image acquisition device 4 is integrated in a portable electronic device of the user (for example, a smartphone or a tablet) or in another electronic device (for example, a console) installed or present (even simply resting inside the training space A or present or integrated in at least one exercise machine 5.

In an embodiment, in combination with any of those described above and shown in Figure 3a, the at least one central data processing unit 1 and the at least one central memory unit 2 are integrated in the at least one image acquisition device 4.

In a further embodiment, alternatively to the preceding one and shown in Figure 3b, the system 100 comprises an electronic computer 20 present inside the training space A.

In this embodiment, the at least one central data processing unit 1 and the at least one central memory unit 2 are integrated in the electronic computer 20.

In this embodiment, the at least one image acquisition device 4 is operatively connected to the central data processing unit 1.

In accordance with different embodiments, in combination with the preceding one, the at least one image acquisition device 4 is operatively connected to the central data processing unit 1 in a wired mode or in a wireless mode.

In an embodiment, the at least one image acquisition device 4 is operatively connected to the central data processing unit 1 by means of the data communication network NTW, already described above.

In a further embodiment, alternatively to or in combination with the preceding one, the at least one image acquisition device 4 is operatively connected to the central data processing unit 1 in a wireless mode by means of a wireless technology data communication channel such as, for example, a Wi-Fi, Bluetooth, NFC data communication channel, and so on.

In this embodiment, the at least one image acquisition device 4 is configured to communicate data with the central data processing unit 1.

In accordance with different embodiments, the data communication between the at least one image acquisition device 4 and the central data processing unit 1 can occur by means of the data communication network NTW and/or a wireless technology data communication channel (Wi-Fi, Bluetooth, NFC, and so on).

In a further embodiment, alternative to those in Figures 3a and 3b, shown in Figure 3c, the system 100 comprises an electronic computer 20 present inside the training space A.

In this embodiment, the at least one central data processing unit 1 is integrated in the at least one image acquisition device 4 and the at least one central memory unit 2 is integrated in the electronic computer 20.

In a further embodiment, alternative to those in Figures 3a and 3b and 3c, shown in Figure 3d, the system 100 comprises an electronic computer 20 present inside the training space A.

In this embodiment, the at least one central data processing unit 1 is integrated in the electronic computer 20 and the at least one central memory unit 2 is integrated in the at least one image acquisition device 4.

In a further embodiment, alternative to those in Figures 3a, 3b, 3c, 3d, shown in Figure 3e, the system 100 comprises an electronic computer 30 which is remote with respect to the training space A, for example a cloud server.

In this embodiment, the at least one central data processing unit 1 is integrated in the at least one image acquisition device 4 and the at least one central memory unit 2 is integrated in the remote electronic computer 30.

In an embodiment, in combination with the preceding one, the at least one image acquisition device 4 is operatively connected to the remote electronic computer 30.

In accordance with different embodiments, the at least one image acquisition device 4 is operatively connected to the remote electronic computer 30 in a wired mode or in a wireless mode.

In an embodiment, the at least one image acquisition device 4 is operatively connected to the remote electronic computer 30 by means of the data communication network NTW, already described above.

In a further embodiment, alternatively to or in combination with the preceding one, the at least one image acquisition device 4 is operatively connected to the remote electronic computer 30 in a wireless mode by means of a wireless technology data communication channel such as, for example, a Wi-Fi, Bluetooth, NFC data communication channel, and so on.

In this embodiment, the at least one image acquisition device 4 is configured to communicate data with the remote electronic computer 30.

In accordance with different embodiments, the data communication between the at least one image acquisition device 4 and the remote electronic computer 30 can occur by means of the data communication network NTW and/or a wireless technology data communication channel (Wi-Fi, Bluetooth, NFC, and so on).

In a further embodiment, alternative to those in Figures 3a, 3b, 3c, 3d and 3e, shown in Figures 3f, the system 100 comprises an electronic computer 20 present inside the training space A and an electronic computer 30 which is remote with respect to the training space A, for example a cloud server.

In this embodiment, the at least one central data processing unit 1 is integrated in the electronic computer 20 and the at least one central memory unit 2 is integrated in the remote electronic computer 30.

In this embodiment, the at least one image acquisition device 4 and the electronic computer 20 are operatively connected to each other and to the remote electronic computer 30.

In accordance with different embodiments, the at least one image acquisition device 4 and the electronic computer 20 are operatively connected to each other and to the remote electronic computer 30 in a wired mode or in a wireless mode.

In an embodiment, the at least one image acquisition device 4 and the electronic computer 20 are operatively connected to one another and to the remote electronic computer 30 by means of the data communication network NTW, already described above.

In a further embodiment, alternatively to or in combination with the preceding one, the at least one image acquisition device 4 and the electronic computer 20 are operatively connected to each other and to the remote electronic computer 30 in a wireless mode by means of a wireless technology data communication channel such as, for example, a Wi-Fi, Bluetooth, NFC data communication channel, and so on.

In this embodiment, the at least one image acquisition device 4 and the electronic computer 20 are configured to communicate data with one another and with the remote electronic computer 30.

In accordance with different embodiments, the data communication between the at least one image acquisition device 4, the electronic computer 20 and the remote electronic computer 30 can occur by means of the data communication network NTW and/or a wireless technology data communication channel (Wi-Fi, Bluetooth, NFC, and so on).

In an embodiment, not shown in the Figures, in combination with any of those described above, the at least one central data processing unit 1 comprises one or more data processing modules distributed on one or more of said at least one image acquisition device 4 and/or the electronic computer 20 present inside the training space A (if present).

In an embodiment, not shown in the Figures, in combination with any of those described above and alternatively to the preceding one, the at least one central data processing unit 1 comprises one or more data processing modules distributed on one or more of said at least one image acquisition device 4 and/or an electronic computer 20 present inside the training space A (if present).

In this embodiment, the at least one central memory unit 2 comprises one or more memory modules distributed on one or more of said at least one image acquisition device 4 and/or on said electronic computer 20 present inside the training space A (if present) and/or on an electronic computer 30 which is remote with respect to training space A (if present).

In accordance with an embodiment, in combination with any of the preceding ones, the set training program comprises a plurality of physical exercises identifiable for each user of said plurality of users P-U.

In greater detail, a plurality of physical exercises is set for each user of said plurality of users P-U in accordance with a set prescription by, for example, a trainer or other training expert.

The physical exercises set (prescribed) for a user are those which the at least one data processing unit 1 will be able to identify from time to time, for that user, during the implementation of the system or the execution of the method described below.

According to a first example shown in Figure 7, in which the plurality of users P-U comprises a first user identifiable by respective user identification code ID1, a second user identifiable by respective user identification code ID2, a third user identifiable by respective user identification code ID3, a fourth user identifiable by respective user identification code ID4, the set training program, indicated with reference P7, comprises a first physical exercise E1, a second physical exercise E2, a third physical exercise E3, a fourth physical exercise E4.

For example, considering for simplicity a round lasting 1 minute:
- the first physical exercise E1 is a treadmill run at 12 km/h;
- the second physical exercise E2 is the performance of squats;
- the third physical exercise E3 is the performance of arm lifts using a barbell;
- the fourth physical exercise E4 is the performance of bicep curls with an elastic band.

In this example, the set training program P7 requires each user to temporally perform in sequence the first physical exercise E1, the second physical exercise E2, the third physical exercise E3, the fourth physical exercise E4, each identified for a user depending on the training round provided by the set training program P7.

In a set first training time interval, the plurality of users P-U are in a first training round R1.

In accordance with the first round of training R1, the set training program P7 includes:
- for the first user ID1, the first physical exercise E1;
- for the second user ID2, the second physical exercise E2;
- for the third user ID3, the third physical exercise E3;
- for the fourth user ID4, the fourth physical exercise E4.

Upon reaching the target provided by each exercise for each user, the at least one data processing unit 1 provides each user with an indication of the next physical exercise provided by the set training program in a subsequent round.

This is valid for each step between consecutive rounds within the sequence of first round R1, second round R2, third round R3, fourth round R4.

Again with reference to the previous example, during the first training round R1, the at least one central data processing unit 1:
- while performing the first physical exercise E1, since the treadmill is configured to detect and communicate data, it receives the training data from the treadmill;
- while performing the second physical exercise E2, since the training is a bodyweight training, it determines the squat repetitions performed by the user from the images acquired by the at least one image acquisition device 4;
- while performing the third physical exercise E3, if the barbell is not configured to detect and communicate data, it determines the repetitions of push-ups with the barbell and the weights loaded on the barbell (for example, by detecting the color or another distinctive sign), from the images acquired by the at least one image acquisition device 4;
- while performing the fourth physical exercise E4, since the elastic is not configured to detect and communicate data, it determines the repetitions of bicep curls and the resistance of the elastic (for example by detecting the color or another distinctive sign), from the images acquired by at least one image acquisition device 4.

Returning now in general to the example in Figure 7, in a set second training time interval, following the first training time interval, the plurality of users P-U is in a second training round R2.

In accordance with the second round of training R2, the set training program P7 includes:
- for the first user ID1, the fourth physical exercise E4;
- for the second user ID2, the first physical exercise E1;
- for the third user ID3, the second physical exercise E2;
- for the fourth user ID4, the third physical exercise E3.

In a set third training time interval, following the second training time interval, the plurality of users P-U is in a third training round R3.

In accordance with the third round of training R3, the set training program P7 includes:
- for the first user ID1, the third physical exercise E3;
- for the second user ID2, the fourth physical exercise E4;
- for the third user ID3, the first physical exercise E1;
- for the fourth user ID4, the second physical exercise E2.

In a set fourth training time interval, following the third training time interval, the plurality of users P-U is in a fourth training round R4.

In accordance with the fourth round of training R4, the set training program P7 includes:
- for the first user ID1, the second physical exercise E2;
- for the second user ID2, the third physical exercise E3;
- for the third user ID3, the fourth physical exercise E4;
- for the fourth user ID4, the first physical exercise E1.

According to a second example shown in Figure 8, in which the plurality of users P-U comprises a first user identifiable by respective user identification code ID1, a second user identifiable by respective user identification code ID2, a third user identifiable by respective user identification code ID3, a fourth user identifiable by respective user identification code ID4, the set training program, indicated with reference P8, always comprises a first physical exercise E1, a second physical exercise E2, a third physical exercise E3, a fourth physical exercise E4.

In this second example, the set training program P8 requires that the first physical exercise E1, the second physical exercise E2, the third physical exercise E3, the fourth physical exercise E4 are identified in sequence for each user depending on the round of training provided by the set training program P8.

In a first round of training R1, the set training program P8 includes:
- the first physical exercise E1 for the first user ID1;
- the second physical exercise E2 for the fourth user ID4;
- the third physical exercise E3 for the third user ID3;
- the fourth physical exercise E4 for the second user ID2.

In a second round of training R2, following the first round of training R1, the set training program P8 includes:
- the first physical exercise E1 for the second user ID2;
- the second physical exercise E2 for the first user ID1;
- the third physical exercise E3 for the fourth user ID4;
- the fourth physical exercise E4 for the third user ID3.

In a third round of training R3, following the second round of training R2, the set training program P8 includes:
- the first physical exercise E1 for the third user ID3;
- the second physical exercise E2 for the second user ID2;
- the third physical exercise E3 for the first user ID1;
- the fourth physical exercise E4 for the fourth user ID4.

In a fourth round of training R4, following the third round of training R3, the set training program P8 includes:
- the first physical exercise E1 for the fourth user ID4;
- the second physical exercise E2 for the third user ID3;
- the third physical exercise E3 for the second user ID2;
- the fourth physical exercise E4 for the first user ID1.

In accordance with an embodiment, in combination with the preceding one, the set training program is stored in the at least one central memory unit 2.

According to an embodiment, in combination with any of the preceding ones, in which the determined value V-TR representative of a training parameter provided by the physical exercise being performed by the user comprises information representative of a count of repetitions performed by the user.

In accordance with an embodiment, in combination with any of the preceding ones, the at least one central data processing unit 1 is configured to determine an information representative of an assessment of the correct performance of the physical exercise by the user based on the determined information I-E representative of training data of the user related to the physical exercise performed by the user.

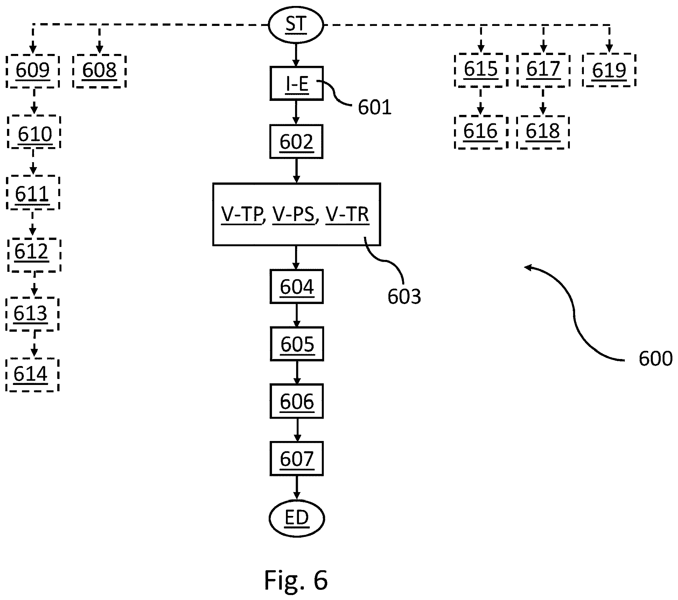

With reference to Figure 6, a method 600 is now described for determining and storing training data of a plurality of users P-U in a training space A, hereinafter also simply a method, according to the present invention.

It is worth noting that the components, data and information mentioned below with the description of the method have already been described previously with reference to the system 100 and will therefore not be repeated for the sake of brevity.

Each user of said plurality of users P-U is assigned with a respective user identification code ID1-IDN.

The user identification code ID1-IDN is stored in at least one central memory unit 2.

The method 600 comprises a symbolic step of starting ST.

The method 600, for each user of said plurality of users P-U, for each current time instant tᵢ, with 1 < i < N, of a plurality of consecutive time instants t₁, t₂, ..., t_{N}, with N being an integer, comprises the following steps.

The method 600 comprises a step of a1) determining 601, by at least one central data processing unit 1, when the user performs the physical exercise identified for the user within a set training program, information I-E representative of training data of the user related to the physical exercise being performed by the user.

The method 600 further comprises a step of b1) storing 602, by said at least one central data processing unit 1, in the at least one central memory unit 2, said information I-E representative of training data of the user related to the physical exercise being performed by the user.

The method 600 further comprises a step of c1) determining 603, by said at least one central data processing unit 1, when the user performs a physical exercise identified for the user within the set training program and stored in the at least one central memory unit 2, at least one of: a value V-TP representative of the time elapsed since the user started performing the physical exercise, a value V-PS representative of the position of the user within the training space A, a value V-TR representative of a training parameter provided by the physical exercise being performed by the user.

The method 600 further comprises a step of d1) identifying 604, by said at least one central data processing unit 1, a next physical exercise for the user to be performed within the training space A provided by the set training program, based on the respective user identification code ID1-IDN and at least one of: whether or not the determined value V-TP representative of the time elapsed since the user started performing the physical exercise reaches a respective time target provided by the physical exercise being performed by the user, the determined value V-PS representative of the position of the user within the training space A, whether or not the determined value V-TR representative of a training parameter provided by the physical exercise being performed by the user reaches a respective training target provided by the physical exercise being performed by the user.

The method 600 further comprises a step of e1) storing 605, by said at least one central data processing unit 1, in the at least one central memory unit 2, the next physical exercise identified for the user.

The method 600 further comprises a step of f1) providing 606 the user, by at least one central data processing unit 1, with an indication of the next physical exercise identified for the user.

If the identification of the next physical exercise occurs based on the fact that the determined value V-TP, representative of the time elapsed since the user started performing the physical exercise, reaches the respective time target provided by the physical exercise performed by the user, the at least one central data processing unit 1 provides the user with the indication of the next physical exercise identified for the user and the user, once aware of such an indication, will reach the station and will actually move on to performing the next physical exercise.

If the identification of the next physical exercise occurs based on the determined value V-PS, representative of the position of the user within the training space A, the at least one central data processing unit 1 provides the user with the indication of the next physical exercise identified for the user. In this case, the user, once aware of such an indication, should already be in the position corresponding to the next identified physical exercise which should also already be performed by the user. If this is not the case, such an indication provided to the user helps the user understand which physical exercise they should actually perform in that position within the training space A.

If the identification of the next physical exercise occurs based on the fact that the determined value V-TR, representative of a training parameter provided by the physical exercise being performed by the user, reaches the respective training target provided by the physical exercise performed by the user, the at least one central data processing unit 1 provides the user with the indication of the next physical exercise identified for the user and the user, once aware of such an indication, will reach the station and will actually move on to performing the next physical exercise.

As mentioned above, in accordance with any of the embodiments described above in which the at least one central data processing unit 1 provides the user with an indication of the next physical exercise identified for the user, such an indication can be provided, for example, by means of a video and/or audio message and/or an update of the status of the training class viewable on a display screen made available to the plurality of users within the training space A.

For example, the state of the training class can be a time diagram (like the one shown in Figure 7) or a matrix (like the one shown in Figure 8) in which each user can intuitively understand, by simply observing the display screen, which physical exercise (E1-E4) has been identified for the user (ID1-ID4) to be performed within a training round (R1-R4) provided by the set training program (P7 in Figure 7; P8 in Figure 8).

The method 600 further comprises a step of g1) repeating 607, by the at least one central data processing unit 1, steps a1) - f1) for the next physical exercise identified for the user.

The method 600 comprises a symbolic step of ending ED.

In accordance with an embodiment, at least one exercise machine 5 is present within the training space A.

The at least one exercise machine 5 comprises at least one actuator 6 which is operable while performing a physical exercise.

The physical exercise identifiable to the user is a physical exercise which can be performed using the at least one exercise machine 5.

In accordance with an embodiment, in combination with any of the preceding ones, the at least one exercise machine 5 is present within the training space A.

The at least one exercise tool 10 is free from any actuator which is operable while performing a physical exercise.

The physical exercise identifiable to the user is a physical exercise which can be performed using the at least one exercise tool 10.

In accordance with an embodiment, in combination with any of the preceding ones, the identified physical exercise being performed by the user involves the use of an exercise machine 5 or exercise tool 10 which is not configured to automatically detect and communicate training data, or in which the identified physical exercise being performed by the user is a bodyweight exercise.

The definition of "exercise machine not configured to detect and automatically communicate training data" and the definition of "exercise tool not configured to detect and automatically communicate training data" have been previously indicated.

In this embodiment, the step of a1) determining 601, when the user performs a physical exercise identified for the user within a set training program, information I-E representative of training data of the user related to the physical exercise being performed by the user is performed by the at least one central data processing unit 1 based on a processing of the images of the user within the training space A acquired by the at least one image acquisition device 4.

As also mentioned above, the images of the user within the training space A acquired by the at least one image acquisition device 4 are a subset of the images of the plurality of users P-U within the training space A acquired by the at least an image acquisition device 4.

In other words, the at least one central data processing unit 1, by processing the images of the plurality of users P-U within the training space A acquired by the at least one image acquisition device 4 is configured to determine the users present within the training space A, recognizing for each of them the respective user identification code ID1-IDN and associating to each of them the respective identified physical exercise as provided by the set training program based on the respective prescription for that specific user identification code.

As it will also be reiterated below, the at least one central data processing unit 1 is capable of processing a diagrammatic model representative of the user (structure with a plurality of key elements (points) such as nodes and segments) and the respective training data (for example, it can count the repetitions performed).

In accordance with an embodiment, in combination with the preceding one, the identified physical exercise being performed by the user involves the use of an exercise machine 5 or exercise tool 10 configured to automatically detect and communicate training data.

In this embodiment, the step of a1) determining 601, when the user performs a physical exercise identified for the user within a set training program, information I-E representative of training data of the user related to the physical exercise being performed by the user is performed by the at least one central data processing unit 1 based on an analysis of the training data received from the exercise machine 5 or the exercise tool 10.

In accordance with an embodiment, in combination with any of the preceding ones, the user identification code ID1-IDN of each user of said plurality of users P-U comprises respective user identification data.

In accordance with an embodiment, in combination with the preceding one and shown with dashed lines in Figure 6, the method 600, for each user of said plurality of users P-U, comprises a step of determining 608, by the at least one central data processing unit 1, from the processing of first user identification images acquired during a first user authentication step, a schematic reference model representative of the user.

Such a schematic reference model comprises a plurality of key elements (points) (nodes and/or segments) with which to uniquely represent the user.

In accordance with an embodiment, in combination with the preceding one and shown with dashed lines in Figure 6, the method 600, for each user of said plurality of users P-U, for each current time instant tᵢ, with 1 < i < N , of the plurality of consecutive time instants t₁, t₂, ..., t_{N}, with N being an integer, comprises a step of determining 609, by the at least one central data processing unit 1, from the processing of the images of the user acquired by the at least one image acquisition device 4, a schematic model representative of the user while performing the identified physical exercise.

Such a schematic model comprises a plurality of key elements (points) (nodes and/or segments) with which to uniquely represent the user.

In this embodiment, the method 600 further comprises a step of associating 610, by the at least one central data processing unit 1, such a schematic model representative of the user while performing the identified physical exercise with the respective user identification code ID1-IDN.

In this embodiment, shown with dashed lines in Figure 6, the method 600, for each user of said plurality of users P-U, for each current time instant t i, with 1 < i < N, of the plurality of consecutive time instants t ₁, t₂, ..., t_{N}, with N being an integer, comprises a step of determining 611, by the at least one central data processing unit 1, the variation over time of the schematic model representative of the user while performing the identified physical exercise.

Such a variation over time of the schematic model representative of the user while performing the identified physical exercise comprises information representative of the movement of the plurality of key elements (points) of the schematic model representative of the user while performing the identified physical exercise.

Moreover, in this embodiment, the method 600 comprises a step of comparing 612, by the at least one central data processing unit 1, the information representative of the movement of the plurality of key elements (points) of the schematic model representative of the user while performing the identified physical exercise with set reference movement patterns stored in the at least one central memory unit 2 relating to the physical exercise identified for the user.

As mentioned above, in the at least one central memory unit 2 a database is stored comprising, for each physical exercise of a plurality of physical exercises identifiable for each user of said plurality of users P-U (which can be set in accordance with a set prescription by, for example, a trainer or other training expert, as it will be reiterated below), a respective set reference movement pattern.

The at least one central data processing unit 1, being the physical exercise identified for the user known, is thus capable of querying the database stored in the at least one central memory unit 2 to recover the respective set reference movement pattern to be used for the aforesaid comparison.

Moreover, in this embodiment, the method 600 comprises a step of providing 613 the user, by means of the at least one central data processing unit 1 (for example, by means of an audio and/or video message), with an information representative of an assessment of the correct performance of the physical exercise by the user based on the outcome of such a comparison.

Therefore, it should be noted that, in this embodiment, the at least one central data processing unit 1 is capable of providing the user with the specific information representative of an assessment of the correct performance of the physical exercise by the user based on the monitoring of the variation over time of the plurality of key elements (points) of the schematic model without having to process any image acquired by the at least one image acquisition device 4, again with a notable saving from a computational point of view.

In this embodiment, shown with dashed lines in Figure 6, the method 600, for each user of said plurality of users P-U, for each current time instant t ᵢ, with 1 < i < N, of the plurality of consecutive time instants t ₁, t₂, ..., t_{N}, with N being an integer, further comprises a step of storing 614, by the at least one central data processing unit 1, in the at least one central memory unit 2, the determined variation over time of the schematic model representative of the user while performing the identified physical exercise.

In accordance with an embodiment, in combination with the preceding one and shown with dashed lines in Figure 6, the method 600, for each user of said plurality of users P-U, for each current time instant tᵢ, with 1 < i < N , of the plurality of consecutive time instants ₁, t₂, ..., t_{N}, with N being an integer, comprises steps of:
- determining 615, by the at least one central data processing unit 1, a power built up by the user while performing the identified physical exercise, based on the images acquired of the user within the training space A, the amplitude and speed of execution of the movements performed by the user determined by the processing of the images acquired by the at least one image acquisition device 4 of the user in the training space A and/or an energy consumed by the user while performing the physical exercise identified for the user;
- associating 616, by the at least one central data processing unit 1, the determined built-up power and/or the determined built-up energy with the set reference movement patterns, if the movements performed by the user during the physical exercise identified for the user correspond to said set reference movement patterns stored in the at least one central memory unit 2.

In accordance with an embodiment, in combination with any of the preceding ones in which the set reference movement patterns are provided and shown with dashed lines in Figure 6, said set reference movement patterns performed by the user comprise movements of at least one exercise tool 10 moved by the user.

In this embodiment, the method 600, for each user of said plurality of users P-U, for each current time instant tᵢ, with 1 < i < N, of the plurality of consecutive time instants t₁, t₂, ..., t_{N}, with N being an integer, comprises steps of:
- determining 617, by the at least one central data processing unit 1, a power built up by the user and/or an energy built up by the user which is a function of the images of the user within the training space A and of said at least one exercise tool 10 while performing the set assigned physical exercise;
- associating 618, by the at least one central data processing unit 1, the determined built-up power and/or the determined built-up energy with the set reference movement patterns, if the movements performed by the user during the set identified physical exercise correspond to set reference movement patterns stored in the at least one central memory unit 2.

In accordance with an embodiment, in combination with any of the preceding ones and shown with dashed lines in Figure 6, a personal device DP is present, wearable by one or more users of said plurality of users P-U.

In this embodiment, the method 600, for each user of said plurality of users P-U, for each current time instant tᵢ, with 1 < i < N, of the plurality of consecutive time instants t₁, t₂, ..., t_{N}, with N being an integer, comprises a step of acquiring 619, by the at least one central data processing unit 1, the user identification code ID1-IDN from the personal device wearable by the user.

According to a further embodiment, in combination with the preceding one, the step of acquiring, for each user of said plurality of users P-U, by the at least one central data processing unit 1, the user identification code ID1- IDN from the personal device wearable by the user, is performed for each current time instant tᵢ, with 1 < i < N, of the plurality of consecutive time instants t₁, t₂, ..., t_{N}, with N being an integer.

With reference to the aforesaid figures, and in particular to the example in Figure 7, an example of operation of the system 100 according to the present invention is described.

A plurality of users P-U (for example, four) are in a training space A.

The first user of said plurality of users P-U is assigned with a respective user identification code ID1.

The second user of said plurality of users P-U is assigned with a respective user identification code ID2.

The third user of said plurality of users P-U is assigned with a respective user identification code ID3.

The fourth user of said plurality of users P-U is assigned with a respective user identification code ID4.

The user identification code of each user is stored in at least one central memory unit 2.

In a set first time interval, corresponding to a first round of training R1, provided by the set training program P7:
- the first user ID1 is performing a first physical exercise E1, for example, a run on a treadmill at 12 km/h;
- the second user ID2 is performing a second physical exercise E2, for example, squats;
- the third user ID3 is performing a third physical exercise E3, for example, push-ups using a barbell;
- the fourth user ID4 is performing a fourth physical exercise E4, for example, bicep curls with an elastic band.

For each user of said plurality of users P-U, for each current time instant tᵢ, with 1 < i < N, of a plurality of consecutive time instants t₁, t₂, ..., t_{N}, with N being an integer, at least one central data processing unit 1 determines, when the user performs the physical exercise identified for the user within the set training program P7, information I-E representative of training data of the user related to the physical exercise being performed by the user.

In more detail, during the first training round R1, the at least one central data processing unit 1:
- while performing the first physical exercise E1, since the treadmill is configured to detect and communicate data, it receives the training data from the treadmill;
- while performing the second physical exercise E2, since the training is a bodyweight training, it determines the squat repetitions performed by the user from the images acquired by the at least one image acquisition device 4;

- while performing the third physical exercise E3, if the barbell is not configured to detect and communicate data, it determines the repetitions of push-ups with the barbell and the weights loaded on the barbell (for example, by detecting the color or another distinctive sign), from the images acquired by the at least one image acquisition device 4;
- while performing the fourth physical exercise E4, since the elastic is not configured to detect and communicate data, it determines the repetitions of bicep curls and the resistance of the elastic (for example by detecting the color or another distinctive sign), from the images acquired by at least one image acquisition device 4.

Again for each user of said plurality of users P-U, for each current time instant tᵢ, with 1 < i < N, of a plurality of consecutive time instants t₁, t₂, ..., t_{N}, with N being an integer, the at least one central data processing unit 1, when the user performs the physical exercise identified for the user within the set training program P7:
- stores, in the at least one central memory unit 2, said information I-E representative of training data of the user related to the physical exercise being performed by the user;
- determines, when the user performs a physical exercise identified for the user within the set training program P7 and stored in the at least one central memory unit 2, a value V-TP representative of the time elapsed since the user started performing the physical exercise;
- identifies a next physical exercise for the user to be performed within the training space A provided by the set training program, based on the respective user identification code ID1-IDN and the achievement by the determined value V-TP representative of the time elapsed since the user started performing the physical exercise of a respective time target provided by the physical exercise being performed by the user.

In particular, for a set second training time interval (corresponding to a second training round R2), following the first training time interval, provided by the set training program P7, the at least one central data processing unit 1 identifies:
- for the first user ID1, the fourth physical exercise E4;
- for the second user ID2, the first physical exercise E1;
- for the third user ID3, the second physical exercise E2;
- for the fourth user ID4, the third physical exercise E3.

For each user of said plurality of users P-U, for each current time instant tᵢ, with 1 < i < N, of a plurality of consecutive time instants t₂, t₁, ..., t_{N}, with N being an integer, the at least one central data processing unit 1:
- stores, in the at least one central memory unit 2, the next physical exercise identified for the user;
- provides the user with an indication of the next physical exercise identified for the user.

Each of the first user ID1, second user ID2, third user ID3, fourth user ID4 thus knows the next physical exercise to perform within the set training program.

The at least one central data processing unit 1 g1) repeats steps a1) - f1) for the next physical exercise identified for the user.

Thereby, for a set third training time interval (corresponding to a third training round R3), following the second training time interval, provided by the set training program P7, the at least one central data processing unit 1 identifies:
- for the first user ID1, the third physical exercise E3;
- for the second user ID2, the fourth physical exercise E4;
- for the third user ID3, the first physical exercise E1;
- for the fourth user ID4, the second physical exercise E2.

In a set fourth training time interval, following the third training time interval, the plurality of users P-U is in a fourth training round R4.

Moreover, by doing so, for a set fourth training time interval (corresponding to a fourth training round R4), following the third training time interval, provided by the set training program P7, the at least one central data processing unit 1 identifies:
- for the first user ID1, the second physical exercise E2;
- for the second user ID2, the third physical exercise E3;
- for the third user ID3, the fourth physical exercise E4;
- for the fourth user ID4, the first physical exercise E1.

As can be seen, the object of the invention is fully achieved.

In fact, the system and method of the present invention allow improving the training experience of a user as much as possible, by tracking and storing training data of a user which is as complete as possible during any exercise performed by a user, whether on or with an exercise machine, bodyweight exercise or using any other equipment available so as to be able to track and store the training data of the user, without necessarily having to resort to the acquisition and processing of digital images acquired by image acquisition devices present within the training space, thus avoiding as much as possible having to resort to onerous and expensive computational resources.

This is achieved by querying set training programs previously stored in the at least one central memory unit based on the user identification code and at least one of: a value representative of the time elapsed since the user started performing the physical exercise, a value representative of the position of the user within the training space, a value representative of a training parameter provided by the physical exercise being performed by the user.

Each of such parameters, in addition to the user identification code, can be obtained from at least one central data processing unit without having to resort to the processing, which is in itself costly from a computational point of view, of digital images of the training space, acquired by image acquisition devices arranged within the training space.

For example, in order to optimize the data processing by the at least one central data processing unit according to the system and the relative method of the invention, a training space, for example inside a gym, can be divided in two distinct areas:
- a first area in which there are only exercise machines and/or exercise tools configured to detect and communicate data. In this case, the at least one central data processing unit is capable of receiving the data communicated by the exercise machines and/or exercise tools and of assigning such data to the respective users, being aware, by the set training program, of the position in which each user is located and/or the next physical exercise identified for each user;
- a second area in which there are only exercise machines and/or exercise tools not configured to detect and communicate data and/or stations for bodyweight training or with free weights. In this case, the at least one central data processing unit is capable of obtaining training data of each user (for example, the count of repetitions performed) from the processing of the images acquired by the at least one image acquisition device present within the training space, and of assigning such data to the respective users.

Those skilled in the art may make changes and adaptations to the embodiments of the system and method described above or can replace elements with others which are functionally equivalent in order to meet contingent needs without departing from the scope of the following claims. Each of the features described as belonging to a possible embodiment can be achieved irrespective of the other embodiments described.

## Claims

1. A system (100) for determining and storing training data of a plurality of users (P-U) in a training space (A), comprising:
- at least one central data processing unit (1);
- at least one central memory unit (2) operatively connected to said at least one data processing unit (1);
- at least one image acquisition device (4) present inside the training space (A) and operatively connected to the at least one central data processing unit (1), said at least one image acquisition device (4) being configured to communicate data with the at least one central data processing unit (1), said at least one image acquisition device (4) being configured to acquire images of said plurality of users (P-U) within the training space (A),
each user of said plurality of users (P-U) being assigned with a respective user identification code (ID1-IDN), said user identification code (ID) being stored in the at least one central memory unit (2);
said at least one central data processing unit (1) being configured to perform, for each user of said plurality of users (P-U), for each current time instant tᵢ, with 1 < i < N, of a plurality of consecutive time instants t₁, t₂, ..., t_{N}, with N being an integer, the following operations:
- a1) determining, when the user performs the physical exercise identified for the user within a set training program, information (I-E) representative of training data of the user related to the physical exercise being performed by the user;
- b1) storing, in the at least one central memory unit (2), said information (I-E) representative of training data of the user related to the physical exercise being performed by the user;
- c1) determining, when the user performs a physical exercise identified for the user within the set training program and stored in the at least one central memory unit (2), at least one of: a value (V-TP) representative of the time elapsed since the user started performing the physical exercise, a value (V-PS) representative of the position of the user within the training space (A), a value (V-TR) representative of a training parameter provided by the physical exercise being performed by the user;
- d1) identifying a next physical exercise for the user to be performed within the training space (A) provided by the set training program, based on the respective user identification code (ID1-IDN) and at least one of: whether or not the determined value (V-TP) representative of the time elapsed since the user started performing the physical exercise reaches a respective time target provided by the physical exercise being performed by the user, the determined value (V-PS) representative of the position of the user within the training space (A), whether or not the determined value (V-TR) representative of a training parameter provided by the physical exercise being performed by the user reaches a respective training target provided by the physical exercise being performed by the user;
- e1) storing, in the at least one central memory unit (2), the next physical exercise identified for the user;
- f1) providing the user with an indication of the next physical exercise identified for the user;
- g1) repeating steps a1) - f1) for the next physical exercise identified for the user.

2. The system (100) according to claim 1, further comprising at least one exercise machine (5) present inside the training space (A), the at least one exercise machine (5) comprising at least one actuator (6) operable while performing a physical exercise, the physical exercise identifiable for the user being a physical exercise which can be performed using the at least one exercise machine (5).

3. The system (100) according to any one of the preceding claims, further comprising at least one exercise tool (10) present inside the training space (A), said at least one exercise tool (10) being devoid of any actuator operable while performing a physical exercise, the physical exercise identifiable for the user being a physical exercise which can be performed using the at least one exercise tool (10).

4. The system (100) according to any one of the preceding claims, wherein the identified physical exercise being performed by the user involves the use of an exercise machine (5) or an exercise tool (10), which is not configured to automatically detect and communicate training data, or wherein the identified physical exercise being performed by the user is a bodyweight exercise, the at least one central data processing unit (1) being configured to determine the information (I-E) representative of training data of the user related to the physical exercise being performed by the user based on a processing of the images of the user within the training space (A) acquired by the at least one image acquisition device (4).

5. The system (100) according to any one of the preceding claims, wherein the identified physical exercise being performed by the user involves the use of an exercise machine (5) or an exercise tool (10), which is configured to automatically detect and communicate training data, the at least one central data processing unit (1) being configured to determine the information (I-E) representative of training data of the user related to the physical exercise being performed by the user based on an analysis of the training data received from the exercise machine (5) or the exercise tool (10).

6. The system (100) according to any one of the preceding claims, wherein the user identification code (ID1-IDN) of each user of said plurality of users (P-U) comprises respective user identification data.

7. The system (100) according to any one of the preceding claims, wherein the at least one central data processing unit (1), for each user of said plurality of users (P-U), for each current time instant tᵢ, with 1 < i < N, of the plurality of consecutive time instants t₁, t₂, ..., t_{N}, with N being an integer, is configured to determine, from a processing of first user identification images acquired during an initial user authentication step, a schematic reference model representative of the user, such a schematic reference model comprising a plurality of key elements with which to uniquely represent the user.

8. The system (100) according to any one of the preceding claims, wherein the at least one central data processing unit (1), for each user of said plurality of users (P-U), for each current time instant tᵢ, with 1 < i < N, of the plurality of consecutive time instants t₁, t₂, ..., t_{N}, with N being an integer, is configured to:
- determine, from the processing of the images of the user acquired by at least one image acquisition device (4), a schematic model representative of the user while performing the identified physical exercise;
- associate such a schematic model representative of the user while performing the identified physical exercise with the respective user identification code (ID1-IDN);
- determine the variation over time of the schematic model representative of the user while performing the identified physical exercise, such a variation over time of the schematic model representative of the user while performing the identified physical exercise comprising information representative of the movement of the plurality of key elements of the schematic model representative of the user while performing the identified physical exercise;
- compare the information representative of the movement of the plurality of key elements of the schematic model representative of the user while performing the identified physical exercise with set reference movement patterns stored in the at least one central memory unit (2) relating to the physical exercise identified for the user;
- provide the user with an information representative of an assessment of the correct performance of the physical exercise by the user based on the outcome of such a comparison;
- store in the at least one central memory unit (2) the determined variation over time of the schematic model representative of the user while performing the identified physical exercise.

9. The system (100) according to any one of the preceding claims, further comprising a personal device (DP) wearable by one or more users of said plurality of users (P-U), the at least one central data processing unit (1), for each user of said plurality of users (P-U), being configured to acquire the user identification code from the personal device (DP) wearable by the user.

10. The system (100) according to any one of the preceding claims, wherein the at least one image acquisition device (4) is installed in the training space (A).

11. The system (100) according to any one of the preceding claims 1 to 10, wherein the at least one image acquisition device (4) is integrated in a portable electronic device of the user or in another electronic apparatus installed or present inside the training space (A) or present or integrated in the at least one exercise machine (5).

12. The system (100) according to any one of the preceding claims, wherein the at least one central data processing unit (1) comprises one or more data processing modules distributed on one or more of said at least one image acquisition device and/or an electronic computer (20) present inside the training space (A).

13. The system (100) according to any one of the preceding claims 1 to 11, wherein the at least one central data processing unit (1) comprises one or more data processing modules distributed on one or more of said at least one image acquisition device and/or an electronic computer (20) present inside the training space (A), the at least one central memory unit (2) comprising one or more memory modules distributed on one or more of said at least one image acquisition device (4) and/or said electronic computer (20) present inside the training space (A) and/or a remote electronic computer (30) from the training space (A).

14. The system (100) according to any one of the preceding claims, wherein the set training program comprises a plurality of physical exercises identifiable for each user of said plurality of users (P-U).

15. A method (600) for determining and storing training data of a plurality of users (P-U) in a training space (A), each user of said plurality of users (P-U) being assigned with a respective user identification code (ID), said user identification code (ID) being stored in at least one central memory unit (2), the method (500), for each user of said plurality of users (P-U), for each current time instant tᵢ, with 1 < i < N, of a plurality of consecutive time instants t₁, t₂, ..., t_{N}, with N being an integer, comprising steps of:
- a1) determining (601), by at least one central data processing unit (1), when the user performs the physical exercise identified for the user within a set training program, information (I-E) representative of training data of the user related to the physical exercise being performed by the user;
- b1) storing (602), by said at least one central data processing unit (1), in the at least one central memory unit (2), said information (I-E) representative of training data of the user related to the physical exercise being performed by the user;
- c1) determining (603), by said at least one central data processing unit (1), when the user performs a physical exercise identified for the user within a set training program and stored in the at least one central memory unit, at least one of: a value (V-TP) representative of the time elapsed since the user started performing the physical exercise, a value (V-PS) representative of the position of the user within the training space (A), a value (V-TR) representative of a training parameter provided by the physical exercise being performed by the user;
- d1) identifying (604), by said at least one central data processing unit (1), a next physical exercise for the user to be performed within the training space (A) provided by the set training program, based on the respective user identification code (ID1-IDN) and at least one of: whether or not the determined value (V-TP) representative of the time elapsed since the user started performing the physical exercise reaches a respective time target provided by the physical exercise being performed by the user, the determined value (V-PS) representative of the position of the user within the training space (A), whether or not the determined value (V-TR) representative of a training parameter provided by the physical exercise being performed by the user reaches a respective training target provided by the physical exercise being performed by the user;
- e1) storing (605), by said at least one central data processing unit (1), in the at least one central memory unit (2), the next physical exercise identified for the user;
- f1) providing (606) the user, by said at least one central data processing unit (1), with an indication of the next physical exercise identified for the user;
- g1) repeating (607), by at least one central data processing unit (1), steps a1) - f1) for the next physical exercise identified for the user.
